# EUROPEAN PATENT APPLICATION

(11) **EP 1 666 049 A1**
(43) Date of publication of application: **07.06.2006**
(21) Application number: 03818011.3
(22) Date of filing: 07.08.2003
(51) Int. Cl.: A61K 35/64, A61K 41/00

(54) **MARKOV THERAPEUTIC AND PREVENTIVE COMPOUND**

(71) Applicant: MARKOV, Gennadij A., Novosibirsk, 630055 (RU)
(72) Inventor: MARKOV, Gennadij A., Novosibirsk, 630055 (RU)
(74) Representative: HOFFMANN EITLE
(86) International application number: PCT/RU2003/000354
(87) International publication number: WO 2005/014019

(57) **Abstract**

The invention relates to the field of medicine and may be used in scientific and practical activity, including the treatment of different human and animal diseases. The compound consists of food products and extracts of therapeutic plants or natural juices as food product solvents. Table salt, sugar and honey, which are used in a determined quantity per 11 of solution and at a specified component ratio, are used as the food products. An herbal plant or herbal tea for which a pharmacological action with respect to a given form of a disease is known is used for producing an extract of therapeutic plants. In order to preserve the compound and prevent the souring thereof, acetic or citric acid is added thereto. In order to accelerate the action of the compound on an organism, it is treated with electric current.

## Description

### Field of the Invention

The instant invention relates to medicine and can be used for scientific and practical activity, including the treatment of different human and animal diseases. A number of requirements are stipulated in respect to such compounds. In particular, they should not provide side effects, should be storable for a lengthy period, preferably at room temperature, should have good taste qualities.

At present the rehabilitation of traditional medicine is on the rise and the only true way to advance is considered to be a combination of two branches of medicine - scientific and traditional. The main part of traditional medicine is phytotherapy. The requirements stipulated in respect to phytotherapy are: 1) the prescription of collections of therapeutic plants making it possible to individualize therapy and 2) the achievement of a rapid clinical effect. It should be noted that a number of illnesses exist, against which pharmacy does not have reliable and safe agents for treatment, for example, malignant tumors of different nature and location. Therefore, the detection of useful properties of the plants and their use for treatment of diseases is a real aim of medicine.

Decoctions are extracts of useful substances of herbs and differ by extragents, such as aqueous, alcoholic and organic extragents. In order to accelerate the process of recovering useful properties from the herbs, the action of an external field, such as ultrasonic, magnetic, electromagnetic, is often used. Recently, methods of preparing therapeutic preparations are being developed, including herbal and homoeopathic, in which the removal of bioinformational properties of the preparation onto a carrier takes place by the action of an electric or magnetic field on the preparation.

### Background of the Art

A method for processing plant feedstock (patent RU 2141327, 6A61K 35/78, published BI 32, 1999) is known comprising pouring boiled water at room temperature onto a plant feedstock and heating it with subsequent cooling of the obtained extract, wherein the heating and cooling are carried out while passing alternating current through the extract.

A method is known for reproducing homoeopathic and isopathetic preparations (patent RU 2074701, 6A611 3/00, published BI 7, 1997), comprising the noncontact transfer of bioinformational properties to an electric or magnetic memory element by acting thereon a constant electric field and a signal adequate to the information properties of the preparation.

A method of obtaining a therapeutic agent is known (patent BE 19520942, 6A61K 41/00, published ISM, v. 8, 22, 1998), in which two substances - a therapeutic agent as an emitter of information and an aqueous solution of a second substance as a receiver of the information - are simultaneously arranged in a static homogeneous magnetic field and an electromagnetic field of high frequency. Wherein, the transmission of information on the therapeutic properties of the emitter substance and information on the receiver substance takes place without contact therebetween.

A compound for the biological stimulation and correction of the phenotype of an organism is known (application WO 94/09797, 5A61K 33/14, 41/00, published 11 May 1994). The compound is a solution of table salt, sugar and honey in water. It comprises mineral additives, such as chlorides of potassium, calcium, magnesium, and also trace elements in the form of chlorides or carbonates of copper, molybdenum, manganese, iron, cobalt, nickel. The compound is subjected to electric current with a pulse length 10⁻⁸÷1 s with a rate of voltage pulse rise of the voltage pulse 1×10⁻⁸÷1×10¹³ V/s, an on-off time ratio (relative pulse duration) of 1÷1×10⁴ and an electrical field intensity between the electrodes 10÷9×10⁴ V/cm. Under this processing, the compound receives information necessary for action on malignant cells, as a result of which the cells are restored. Wherein, the electrical pulses may be preliminarily converted into magnetic, electromagnetic, light or audio pulses.

The compound may comprise instead of table salt an aqueous solution of sea salt in an amount of 1-400 g/l of the solution and sugar 1-3000 g/l, and also be processed with an external source of energy.

Honey in an amount of 1-6000 g/l of the solution is additionally introduced into the compound. Mineral additives, such as chlorides or carbonates of alkaline metal, for example, potassium chloride or carbonate in an amount of 1-320 g/l of the solution, are additionally introduced into the compound. Mineral additives in the form of alkaline earth metal chlorides or carbonates, for example, a chloride or carbonate of calcium in an amount of 1-80 g/l or of magnesium in an amount of 1-5 g/l, are additionally introduced into the compound.

Furthermore, trace elements may additionally be introduced into the compound in the form of chlorides or carbonates of copper, molybdenum, manganese, iron, cobalt, nickel in an amount of 1-4 g/l.

Limitation of the set of components in the preparation or of the method of processing the preparation is a drawback of the known compounds and preparations. A drawback of the known therapeutic agents is also the use of mineral additives and trace elements in the form of chemical preparations, which do not always act favorably on healthy cells of an organism (side effect).

### Summary of the Invention

The object at the base of the invention is to create such a compound for treatment, which would provide a possibility for treatment of a number of diseases, including malignant neoplasms, and would act on those organs or tissues of the organism in need of treatment.

The object of creating a compound free of a negative side effect on the organism was to replace mineral additives and trace elements in the form of chemical preparations with natural plant feedstock.

The stated object is resolved in that a compound of therapeutic-prophylactic action is provided comprising table salt, sugar and honey, which additionally comprises an extract of therapeutic plants in the form of a decoction or extract with the following ratio of the components, g:

| | |
|---|---|
| table salt | 1-400 |
| sugar | 1-3000 |
| honey | 1-6000 |
| decoction or extract of therapeutic plants | 11 |

The stated object is also resolved in that a compound of therapeutic-prophylactic action is provided comprising table salt, sugar and honey, which additionally comprises fruit, berry, vegetable or herb natural juice with the following ratio of components, g:

| | |
|---|---|
| table salt | 1-400 |
| sugar | 1-3000 |
| honey | 1-6000 |
| fruit, berry, vegetable, herb natural juice | 11 |

The stated object is also resolved in that the compound as therapeutic feedstock is provided comprising a herbal plant or a herbal tea corresponding to a known pharmacological action against a given form of a disease.

The stated object is also resolved in that the compound obtained after dissolution of food components in natural juice is a solution from a concentrated to a 2-5% concentration.

The stated object is resolved in that glacial acetic acid in an amount of 1-3.0 g per 11 of solution is additionally introduced into the compound for conservation.

The stated object is resolved in that citric acid in an amount to 30 g per 11 of solution is additionally introduced into the compound for conservation.

The stated object is also resolved in that the compound is treated with electric current having a pulse length of 1×10⁻⁸÷1 s, with the rate of voltage pulse rise 1×10⁻⁸÷1×10¹³ V/s, the on-off time ratio of 1÷1×10⁴ and an electrical field intensity between the electrodes 10÷9×10⁴ V/cm.

The selection of food products comprising the basis of the compound is due to the following.

The selection of table salt, that is sodium chloride, is due to the physicochemical properties of the sodium metal, which plays an important part in live organisms, wherein it has been established that assimilation of sodium in the organism from a solution of salt and sugar takes place more effectively than from medical preparations. It is also known that honey comprises a large number of elements of animate nature, useful for the organism. Furthermore, the introduction of honey into the compound improves the taste qualities and increases the density of the entered information.

The quantitative content of food products within the compound is due to the following reasons: the upper limit of table salt and sugar is established by their solubility in water. The amount of honey is established by the formation of a homogeneous solution with a mixture of salt and sugar in an aqueous extract or in natural juice. In order to dissolve the food products, the use of plant feedstock is proposed. An aqueous extract of therapeutic plants in the form of a decoction or extract or natural plant juice, as a source of complex organic components and mineral substances (such as potassium, magnesium, calcium) and trace elements is used for replacement of these substances when obtained by a chemical way and used as additives. The use of iron-rich plants, and also plants promoting assimilation of iron fill the deficiency of iron in the organism. Furthermore, mainly together with vegetal food a person receives the whole balanced set of microelements which he needs (manganese, zinc, copper, cobalt, fluorine, iodine, chrome, selenium, vanadium, molybdenum and others). Therefore, the replacement of mineral additives and microelements of chemical origin with the same of plant origin is natural and more closely corresponds to human nature.

During the selection of concrete plant feedstock for the compound, information on the pharmacological purpose of the plant was used, i.e., its action on the organism in the case of some disease or other, and therefore the variety of compounds on the basis of the presented formula is broad.

The advantage of the use of an aqueous extract of medicinal plants or natural juices as solvents consists in the following. In case of dissolution of food products (salt, sugar, honey) in pure water in order to give the compound therapeutic properties it should be subjected to complex treatment with electric current to form complex polymer macromolecules or to form certain metastable structures of compounds having therapeutic-prophylactic properties. In the plant feedstock (aqueous extract or natural juice), which is used as a solvent, there is initially a set of complex organic components, and therefore the process of treating the compound in order to impart it with the necessary therapeutic-prophylactic properties is simplified. Furthermore, in the case of administering vegetal feedstock, a slower removal of mineral substances and microelements from the organism takes place, and therefore there is less need for them than in the case of pharmaceutical preparations. In the case of necessity to enhance the useful properties of the vegetal feedstock and reduce undesirable side effects of the plants, treatment of the compound with electric current is carried out. In that case, replenishment of the deficiency of mineral substances takes place faster than in the case of administration of pharmaceutical preparations. The conditions of treatment are selected to be relatively broad and correspond to the problems resolved in each concrete case.

Sometimes in order to accelerate the dissolution of the substances in the solution, the decoction or extract is heated, but without bringing it to the boiling point.

### Variants of Carrying Out the Invention

A method of preparing the compound consists in the following in the case of an aqueous extract of medicinal plants. In accordance with the pharmacological action, a herbal plant is selected or herbal tea is composed, then a decoction of these herbs is prepared with heating (with boiling in the case of using root parts of the herb), in accordance with known recommendations. The obtained aqueous extract is filtered from herb residues and the food components - table salt, sugar and honey - are dissolved therein. After dissolution, 35% acetic acid or crystalline citric acid is added to the compound to prevent souring of the compound. The compound is ready for use. Sometimes, natural juice, such as fruit, berry, vegetable or herbal, is used in accordance with its pharmacological properties instead of an aqueous extract. In that case, the compound is used either concentrated or prior to use is brought with water to any concentration (but not less than 2-5%).

When it is necessary to accelerate the action of the compound on the organism, the compound is treated with electric current. In that case the order of the action is the following. Food components - table salt, sugar and honey - are dissolved in a prepared aqueous extract or in natural juice, the obtained solution is treated with electric current by immersion of electrodes into the solution. The conditions for treatment are within the following limits: pulse length 1×10⁻⁸÷1 s, the rate of voltage pulse rise 10⁻⁸÷10¹³ V/s, the on-off time of 1÷1×10⁴, the electrical field intensity between the electrodes 10÷9×10⁴ V/cm. However, in view of the fact that dissolution of the food component is not carried out in pure water, but rather in a vegetal solvent, complex boundary treatment is not required, it being possible to use only the lower parameters.

Dosage of the compound (in accordance with the Ministry of Health certificate for the Markov phytococktail).

For prophylaxis: 1-2 teaspoons based on 10 kg of weight, 500 ml of the compound for one course of treatment.

For treatment, the dosage is increased: at first not less than 2-3 teaspoons based on 10 kg of weight, 500 ml of the compound for one course of treatment.

The practical applicability of the compound is proven by the present examples.

Example 1. Dry therapeutic calendula (flowers and leaves) in an amount of 80 g is taken, water in an amount of 320 g is poured thereon and an aqueous extract of calendula is prepared while heating, but not to the boiling point. Then the extract is filtered from the plants, brought to a volume of 1 I with water, then 300 g of table salt, 1000 g of sugar are dissolved therein, and 6000 g of honey are dissolved in the obtained compound.

Then 10 g of 35% acetic acid are added to the compound. Before use, the compound is diluted with water to a 5% concentration of the compound in water. The obtained compound has anti-inflammatory and immunomodulating properties.

Example 2. 10 g of table salt, 100 g of sugar and 10 g of honey are dissolved in 1 1 of tomato juice. During the dissolution of these substances in tomato juice, macromolecules are formed that have therapeutic action in the case of dysbacteriosis, after food poisoning and that increase stomach secretion. In order to provide for lengthy storage, 10 g of 35% acetic acid are added to the compound. Before use, the compound is diluted with water to a 5% concentration of the compound in water.

Example 3. 400 g of table salt, 3000 g of sugar are dissolved in 11 of sea buckthorn juice to the formation of a saturated solution, then the solution is heated over low heat to complete dissolution, without allowing boiling of the solution to occur. Before being used, the compound is diluted with water to a 10% concentration.

Example 4. Juice from the burdock stems and leaves is pressed out by passing the burdock through a meat grinder. 5 g of table salt, 70 g of sugar and 500 g of honey are dissolved in 1 I of the juice. Then electrodes are placed in the juice and electric current is switched on. The conditions for processing are the following: pulse length 1×10⁻⁸ s, the rate of voltage pulse rise 1×10¹³ V/s, the on-off time 10, the electrical field intensity between the electrodes 10 V/cm. 20 g of 35% acetic acid is added to the obtained compound, before use the compound is diluted with water to a concentration of 5% compound. The phytococktail has sudorific, choleretic, immunomodulating and general strengthening properties.

Example 5. Markov phytococktail 1. An extract of black tea is prepared with 24 g of tea in 322 ml (g) of water. The extract is filtered from the tea, 3 g of natural instant coffee, 440 g of granulated sugar, 137 g of table salt, 100 g of natural honey are dissolved in the obtained extract, and as a result 1 kg of phytococktail is obtained. This phytococktail is prescribed at the beginning of a course of treatment of any illness, since it restores the balance between the organs and systems of the whole organism. The phytococktail has anti-inflammatory and antiviral properties. It is recommended for stabilization of blood pressure, to reduce allergic tendencies of the organism, increase immunity, accelerate rehabilitation in the pre- and post-operation period.

Example 6. Markov phytococktail 4. 80 g of a dry collection comprising 40 g of John's-wood (H. perforatum) and 40 g of ordinary milfoil, are taken, covered with 20 g of water and the decoction is prepared while heating, but not to the boiling point. Then the obtained decoction is filtered from the remains of the herbs. Food components are dissolved in the obtained decoction: table salt 50 g, granulated sugar 620 g and honey 300 g. The obtained compound is treated with electric current by immersion in a solution with electrodes. The parameters of the treatment are the following: pulse length 3-10⁻⁷ s, the rate of voltage pulse rise 8×10⁶ V/s, the on-off time 18, the electrical field intensity between the electrodes 7.3×10⁴ V/cm. After treatment, 10 g of 35% acetic acid is added to the compound. The dosage of the obtained compound upon administration is 1 teaspoon per 10 kg of weight. The obtained compound has the following therapeutic properties: anti-inflammatory, antimicrobial, hemostatic, promotes normalization of the action of the gastrointestinal tract. Recommended in the case of acute and subacute chronic infectious, chronic diseases of the female genital region, internal bleeding.

Patient E. 36 years old has gastroduodenitis from childhood analysis, and a stomach ulcer from the age of 12, constant recurrence, remission 1-2 months, ulcerous bleeding twice, inpatient treatment twice a year. 5 courses of treatment with the Markov phytococktail 4 in combination with the use of Markov phytococktail 1 and 3 were carried out. The result: there was not a single exacerbation from the moment of beginning treatment with phytococktails, the patient did not get hospital treatment even once.

Example 7. Markov phytococktail 5. 80 g of a dry collection comprising peony (P. anomala) (30 g) and burdock (50 g), 320 g of water are taken, boiled until a decoction is obtained, then the obtained decoction is filtered and food components in the following amounts are dissolved therein: table salt 50 g, granulated sugar 620 g, honey 50 g. The obtained compound is treated with electric current by immersion of electrodes therein. The treatment parameters are the following: pulse length 1×10⁻³ s, the rate of voltage pulse rise 1×10⁷ V/s, the on-off time 1×10³, the electrical field intensity between the electrodes 1×10⁴ V/cm. After treatment 10 g of 35% acetic acid are added to the compound to conserve the compound - prevent souring. The dosage during administration is 1 teaspoon per 10 kg weight. The obtained compound has anti-inflammatory action, is used for treatment of infectious, gynecological, oncological (in particular brain tumors) diseases, and also for normalization of action of the gastrointestinal tract.

Female patient K. came with a diagnosis of oophoritis, commissural process in the small pelvis, sterility for 8 years. Complaints on the irregularity of the menstrual cycle, strong pain in the lower abdomen during the menstrual cycle. 4 courses of treatment with Markov phytococktails 5 and 3 courses in combination with phytococktails 1, 3, 4 were carried out. The result - after 5 courses the menstrual cycle normalized, pain disappeared, there was no exacerbation of oophoritis. She became pregnant 1.5 years after the beginning of treatment.

Example 8. The following are dissolved in 11 of apple-carrot natural juice (taken in a 1:1 ratio): table salt 100 g, granulated sugar 2400 g, honey 900 g, and the obtained compound is treated with electric current by immersion of electrodes with the following parameters: pulse length 1×10⁻¹ s, the rate of voltage pulse rise 8×10⁴ V/s, the on-off time 2×10², the electrical field intensity between the electrodes 1×10³ V/cm. After treatment, 10 g of citric acid are added to the compound to prevent souring of the compound. Before use, the compound was diluted to a 10% concentration. The dosage of the compound during administration is 2 teaspoons per 10 kg weight. The compound has hematopoietic properties, is used for restoring the blood formula, as an antitumor agent and for normalization of the activity of the gastrointestinal tract.

Patient: a child 3.5 years old. Complaints of frequent colds monthly during 1.5 years. Two times acute bronchitis, whooping cough. During treatment with phytococktail (6 courses) was sick with viral ARD twice. During the illness took phytococktail. The main symptoms of the disease were mixed: a cold, sneezing, hoarse voice.

Thus, the difference of the vegetative feedstock, the broadness of the treatment parameters and the quantitative content of the food filler make it possible to create a large variety of compounds of treatment - prophylactic action.

## Claims

1. A Markov compound of therapeutic-prophylactic action, comprising table salt, sugar and honey, **characterized in that** it additionally comprises an extract of therapeutic plants in the form of a decoction or extract with the following ratio of the components, g:
| | |
|---|---|
| table salt | 1-400 |
| sugar | 1-3000 |
| honey | 1-6000 |
| decoction or extract of therapeutic plants | 11 |

2. A Markov compound of therapeutic-prophylactic action, comprising table salt, sugar and honey, **characterized in that** it additionally comprises fruit, berry, vegetable or herb natural juice with the following ratio of components, g:
| | |
|---|---|
| table salt | 1-400 |
| sugar | 1-3000 |
| honey | 1-6000 |
| fruit, berry, vegetable, herb natural juice | 11 |

3. The compound according to claim 1, **characterized in that** it comprises as plant feedstock a herbal plant or a herbal tea in accordance with its known pharmacological action against a given form of a disease.

4. The compound according to claim 2, **characterized in that** it is a solution from a concentrated to a 2-5% concentration.

5. The compound according to any one of claims 1-4, **characterized in that** acetic acid in an amount of 1-3.0 g per 11 of solution is introduced therein.

6. The compound according to any one of claims 1-4, **characterized in that** crystalline citric acid in an amount to 30 g per 11 of solution is introduced therein.

7. The compound according to any one of claims 1-3, **characterized in that** it is treated with electric current having a pulse length of 1×10⁻⁸÷1 s, a rate of voltage pulse rise of 1×10⁻⁸÷1×10¹³ V/s, an on-off time ratio of 1÷1×10⁴ and an electrical field intensity between the electrodes of 10÷9×10⁴ V/cm.
